# NEUE EUROPÄISCHE PATENTSCHRIFT

(11) **EP 0 620 024 B2**
(45) Veröffentlichungstag und Bekanntmachung des Hinweises auf die Entscheidung über den Einspruch: **25.11.2009**
(45) Hinweis auf die Patenterteilung: 12.03.1997
(21) Anmeldenummer: 94100796.5
(22) Anmeldetag: 20.01.1994
(51) Int. Cl.: A61N 1/05

(54) **Elektrodenvorrichtung**
Electrode device
Dispositif d'électrodes

(30) Priorität: 12.02.1993 SE 9300469
(43) Veröffentlichungstag der Anmeldung: 19.10.1994
(73) Patentinhaber: St. Jude Medical AB, 175 84 Järfälla (SE)
(72) Erfinder: Lindegren, Ulf, S-122 35 Enskede (SE); Petersson, Mats, S-112 31 Stockholm (SE)
(74) Vertreter: Gray, Helen Mary

(56) Entgegenhaltungen:
- WO-A-93/00130
- US-A- 3 911 928
- US-A- 4 649 937
- US-A- 4 848 352
- US-A- 4 955 382
- US-A- 5 097 843

## Beschreibung

Die Erfindung bezieht sich auf eine Elektrodenvorrichtung gemäß dem Oberbegriff des Patentanspruchs 1.

Eine solche Elektrodenvorrichtung ist durch die US-PS 3 911928 bekannt. Der Kopf der Elektrodenvonichtung ist mit einer Anzahl verhältnismässig kleiner, leitender Oberflächen versehen, damit der Schwellenwert und damit auch der Energieverbrauch auf diese Weise gesenkt wird. Der Elektrodenkopf besteht aus einem Kern aus einem elektrisch leitenden Material mit vom Kern herausragenden, vorgeformten Teilen, wobei der Zwischenraum zwischen diesen herausragenden Teilen mit einem elektrisch isolierenden Material ausgefüllt ist. Die vorgeformten herausragenden Teile können streifenförmig, dornenförmig odgl. ausgebildet sein, damit am Elektrodenkopf eine streifenförmige oder punktförmige Verteilung der Stimulationsoberflache erhalten wird. Ein solcher Aufbau des Elektrodenkopfes ist herstellungsmässig kompliziert und dadurch teuer.

Bei einer Elektrodenvorrichtung der eingangs genannten Art, die einen Elektrodenkopf, der eine Anzahl kleine Stimulationsoberflächen hat, aufweist, ist es erforderlich, dass diese Oberflächen einen guten Kontakt zum stimulierbaren Herzgewebe haben. Daher ist es notwendig, dass das Isokliermterial, das einen grossen Teil der Oberfläche des Elektrodenkopfes bildet, extrem biokompatibel ist, damit das Risiko einer fibrösen Gewebebildung um den Elektrodenkopf reduziert wird. Die Gefahr fibrösen Gewebes ist, dass es so dick um den Elektrodenkopf wachsen kann, dass es zu einer Vergrösserung des Abstandes zwischen den Stimulationsoberflächen und dem stimulierbaren Herzgewebe kommt.

Der Erfindung liegt die Aufgabe zugrunde, eine Elektrodenvorrichtung der eingangs genannten Art mit einem Elektrodenkopf zu schaffen, der im Aufbau einfach und daher verhältnismässig billig ist und bei dem gewährleistet ist, dass dessen Stimulationsoberflächen bei einer implantierten Elektrodenvorrichtung einen sehr guten Kontakt mit dem zu slimulierenden Herzgewebe hat. Ausserdem soll das Isoliermaterial extrem biokompatibel sein.

Diese Aufgabe ist erfindungsgemäss durch die Merkmale des Patentanspruchs 1 gelöst. Dadurch, daß die Oberflächenschicht des Elektrodenkopfes mit einem dünnen hochohmigen Isoliermaterial bedeckt wird, können die Herstellungskosten bezüglich dieses Teils der Elektrodenvorrichtung erheblich reduziert werden. Auch die Form und die Grösse der Stimulationsoberflächen können durch die Erfindung in einer einfachen und gewünschten Weise variiert werden. In einem Artikel in der Fachzeitschrift "Journal of Surgical Research". Band 11, Nr. 3, Mars 1971, Seiten 105 - 110 mit dem Titel "Decreasing electrode size and increasing efficiency of Cardiac stimulation" von Furman, Parker und Escher ist das Verhältnis zwischen der Grösse der Stimulationsoberfläche und dem Schwellenwert beschrieben. In einem hier gezeigten Diagramm kann abgelesen werden, dass der Schwellenwert proportional zu einer kleiner werdenden Grösse der Stimulationsoberfläche gesenkt wird. In dem gleichen Artikel ist ein Elektrodenkopf für eine Herzschrittmacherelektrode von einem sog. Kugelkopf-Modell abgebildet und beschrieben. Der Kugelkopf, der als Stimulationsoberfläche dient, ist derart klein, dass diese Elektrode zu den kleinflächigen Elektroden gezählt wird. Der Nachteil einer solchen Form und Grösse des Elektrodenkopfes ist, dass er leicht die Herzwand beschädigen und im schlimmsten Fall penetrieren kann.

Eine Vergrösserung des Abstandes zwischen der Stimulationsoberfläche und einem stimulierbaren Herzgewebe mit 0,1 mm kann den Schwellenwert um etwa 0,5 V erhöhen. In diesem Zusammenhang kann erwähnt werden, dass der Durchschnitt des Schwellenwertes einer Stimulationsoberfläche mit einer Flächengrösse von 3,5 mm² etwa 0,6 V ist. Je kleiner die Stimulationsfläche, um so wesentlicher ist es, dass der Abstand klein ist. Daher liegt nach der Erfindung die Stärke des Isoliermateriales zwischen 0,1 und 10 µm. Eine derartig dünne Isoliermaterialschicht hat weder Einfluss auf den Schwellenwert noch auf die Form des Elektrodenkopfes.

In einer vorteilhaften Ausbildung der Erfindung wird vorgeschlagen, dass das Isoliermaterial aus einem extremen harten Kohlenstoff, auch "diamond like carbon" mit der Verkürzung DLC genannt, besteht. Dieses Material kann sehr dünn aufgetragen werden und trotzdem als ein hochohmiges Isoliermaterial dienen. Das Isoliermaterial ist ferner extrem biokompatibel. Dadurch, dass das Material auch extrem hart ist, wird die isolierende Oberfläche am Elektrodenkopf sehr abreibfest. In der Fachzeitschrift "Diamond and related materials" von 1992 ist auf Seite 727 bis 773 ein Artikel über das Material DLC publiziert. Das Isoliermaterial DLC kann u.a. mit Hilfe von Laser aufgelegt werden. In der schwedischen Fachzeitschrift "Ytform" Nr. 6, Seite 19 von 1992 ist beschrieben, dass mit einer chemischen Reaktion, die durch einen dünnen Laserstrahl ausgelöst wird, dünne Flächenbelege in komplizierten Mustern geschaffen werden können, wobei bestimmte Teile, die als Stimulationsoberflächen vorgesehen sind, nicht mit der DLC-Schicht belegt werden. Eine andere Art, einen Elektrodenkopf des genannten Typs zu erhalten ist, die gesamte Oberfläche des Kopfes mit einer DLC-Schicht zu belegen und danach die Stimulationsoberflächen mittels Fotoätzung nach Wunsch freizulegen. Der Hersteller kann also mittels Laser oder Fotoätzung entscheiden, wie der DLC-Belag Ober die Elektrodenfläche verteilt werden soll. Dadurch wird ein gewünschtes Muster erhalten.

Die Erfindung ist nachfolgend anhand eines in den Zeichnungen dargestellten Ausführungsbeispiels naher erläutert. Es zeigen:

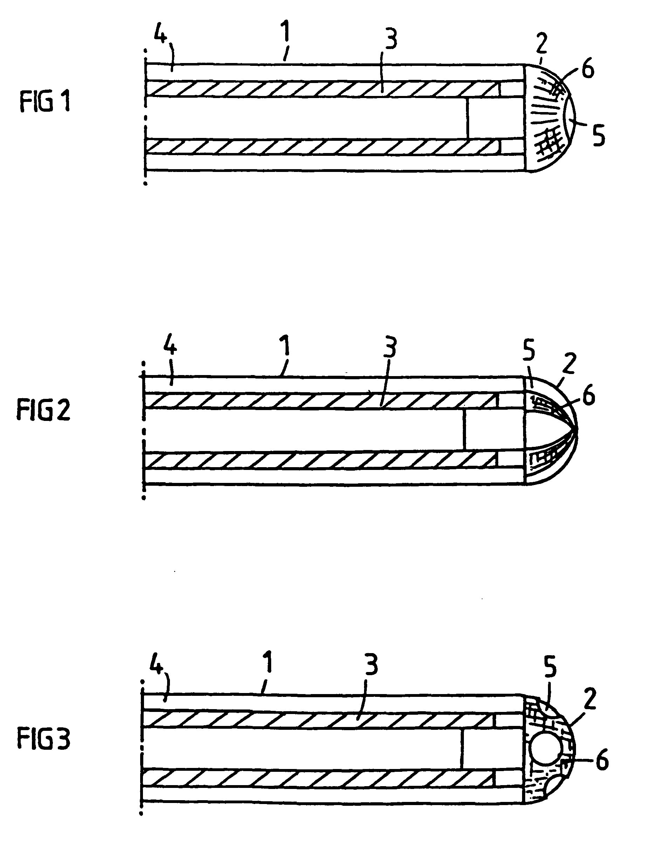

- FIG 1 bis 3: Seitenansichten des distalen Endes einer Elek trodenvorrichtung nach der Erfindung mit ver schiedenen Ausfüh- rungsformen der Stimulations- bzw. Isolieroberflächen am Elektrodenkopt.

In der FIG 1 ist das distale Ende einer Elektrodenvorrichtung zur intrakardialen Stimulation von Herzgewebe eines Patienten abgebildet. Die Elektrodenvorrichtung umfasst ein Elektrodenkabel 1, an dessem distalen Ende ein Elektrodenkopf angebracht ist. Der Elektrodenkopf 2 besteht aus einem elektrisch leitenden Material, wie z.B. Titannitrid und ist an einem Ianggestreckten Leiter 3 angeschlossen, der sich bis zu dem hier nicht gezeigten proximalen Ende des Elektrodenkabels erstreckt. Das Elektrodenkabel 1 ist auch mit einer äusseren Isolierschicht 4 versehen. In dieser FIG ist gezeigt, dass der Elektrodenkopf 2 mit einer zentrisch angeordneten runden Stimulationsoberfläche 5 versehen ist. Die übrige Oberflächenschicht des Elektrodenkopfes ist mit einem Isoliermaterial 6, das "diamond like carbon" genannt und als DLC verkürzt wird, versehen. Dieses Material ist hochohmig, extrem hart und ausserdem extrem biokompatibel. Das Material kann derart dünn aufgelegt werden, dass der Unterschied des Abstandes zwischen der Stimulationsoberfläche und dem Herzgewebe bzw. der Isolierschicht und dem Herzgewebe bei einer applizierten Elektrodenvorrichtung den Schwellenwert nicht beeinflusst. Mit anderen Worten wird die Form des Elektrodenkopfes durch den Belag nicht beeinflusst.

In der FIG 2 ist der Elektrodenkopf 2 in dreieckförmige Abschnitte 5, 6 aufgeteilt, bei denen jeder zweite Abschnitt 6 mit einer Schicht des erwähnten Isoliermaterials bedeckt ist, wobei die übrigen Abschnitte 5 als Stimulationsoberflächen dienen.

In der FIG 3 ist der Elektrodenkopf 2 mit einer Anzahl runder Stimulationsoberflächen 5, die mittels einer Schicht 6 aus einem DLC-Material voneinander Isoliert sind, versehen.

Der Elektrodenkopf der Elektrodenvorrichtung nach der Erfindung ist nicht auf die beschriebenen und gezeigten Ausführungsformen beschränkt. Das Wesentliche ist, dass durch die Erfindung ein in der Herstellung einfacher und billiger Elektrodenkopf gegeben ist, an dem ein Isoliermaterial mit den erwähnten guten Eigenschaften in einer einfachen Weise derart Ober die Oberfläche des Elektrodenkopfes verteilt werden kann, dass ein gewünschtes Muster mit einer gewünschten Anzahl Stimulationsoberflächen, deren Grösseren und Formen variiert werden könnten, erhalten wird.

## Patentansprüche

1. Elektrodenvorrichtung zur intrakorporalen Stimulation des Körpergewebes, insbesondere zur intrakardialen Stimulation von Herzgewebe mit einem Elektrodenkabel (1), das mindestens einen langgestreckten isolierten Leiter (3) und einen Elektrodenkopf (2), der am distalen Ende des Elektrodenkabels (1) angebracht ist, umfasst, wobei die Oberfläche des Elektrodenkopfes (2) teils aus Isoliermaterial (6) und teils aus einem mit dem Leiter verbundenen elektrisch leitenden Material, das mindestens eine Stimulationsoberfläche (5) bildet, besteht, wobei mindestens die gesamte Oberflächenschicht des Elektrodenkopfes (2) aus einem leitenden Material besteht und wobei diese Oberflächenschicht die gewünschte Form des Elektrodenkopfes definiert und wobei das leitende Material teilweise mit einer Schicht aus einem hochohmigen Isoliermaterial (6) bedeckt ist, die derart dünn ist, dass der Unterschied des Abstandes zwischen der Stimulationsoberfläche und dem Herzgewebe bzw. der Isolierschicht (6) und dem Herzgewebe bei einer applizierten Elektrodenvorrichtung den Schwellenwert nicht beeinflusst **dadurch gekennzeichnet, dass** die Stärke des Isoliermaterials (6) zwischen 0,1 und 10 µm ist.

2. Elektrodenvorrichtung nach Anspruch 1, **dadurch gekennzeichnet, dass** das Isoliermaterial (6) aus einer extrem harten Kohlenstoff, auch "diamond like carbon" genannt, besteht.

## Claims

1. Electrode device for intra-body stimulation of the body tissue, in particular for intracardial stimulation of heart tissue, with an electrode cable (1) which comprises at least one elongated insulated conductor (3) and an electrode head (2) which is fitted to the distal end of the electrode cable (1), wherein the surface of the electrode head (2) consists in part of insulating material (6) and in part of an electrically conductive material that is connected to the conductor, which electrically conductive material forms at least one stimulation surface (5), wherein at least the entire surface layer of the electrode head (2) consists of a conductive material and wherein this surface layer defines the desired form of the electrode head and wherein the conductive material is partially covered with a layer of a high-resistance insulating material (6) which is of such a thinness that the difference in the distance between the stimulation surface and the heart tissue and the distance between the insulating layer (6) and the heart tissue does not influence the threshold value with an applied electrode device,
**characterized in that** the thickness of the insulating material (6) is between 0.1 and 10 µm.

2. Electrode device according to claim 1,
**characterized in that** the insulating material (6) consists of an extremely hard carbon, also called "diamond-like" carbon.

## Revendications

1. Dispositif à électrode pour la stimulation intracorporelle du tissu du corps, notamment pour la stimulation intracardiaque du tissu du coeur, comportant un câble (1) à électrode, qui comprend au moins un conducteur (3) isolé s'étendant en longueur et une tête (2) d'électrode montée à l'extrémité distale du câble (1) à électrode, la surface de la tête (2) d'électrode étant en partie en un matériau isolant (6) et en partie en un matériau conducteur de l'électricité qui est relié au conducteur et qui forme au moins une surface (5) de stimulation, au moins l'ensemble de la couche superficielle de la tête (2) d'électrode étant en un matériau conducteur et cette couche superficielle définissant la forme souhaitée de la tête d'électrode et le matériau conducteur étant recouvert en partie d'une couche en un matériau isolant (6) de grande valeur ohmique qui est mince de telle sorte que la différence de distance entre la surface de stimulation et le tissu du coeur ou entre la couche isolante (6) et le tissu du coeur n'influence pas la valeur de seuil lorsque le dispositif à électrode est appliqué, **caractérisé en ce que** l'épaisseur du matériau (6) isolant est comprise entre 0,1 et 10 µm.

2. Dispositif à électrode suivant la revendication 1, **caractérisé en ce que** le matériau isolant (6) est en un carbone extrêmement dur, également appelé "diamond-like carbon".
